# EUROPEAN PATENT SPECIFICATION

(11) **EP 2 755 990 B1**
(45) Date of publication and mention of the grant of the patent: **20.07.2016**
(21) Application number: 12832504.0
(22) Date of filing: 07.09.2012
(51) Int. Cl.: C07J 63/00

(54) **METHOD FOR PREPARATION OF BETULINIC ACID**
VERFAHREN ZUR HERSTELLUNG VON BETULINSÄURE
PROCÉDÉ DE PRÉPARATION D'ACIDE BÉTULINIQUE

(30) Priority: 12.09.2011 SE 1150818
(43) Date of publication of application: 23.07.2014
(73) Proprietor: Stora Enso Oyj, 00101 Helsinki (FI); Koskimies, Salme, 00850 Helsinki (FI)
(72) Inventor: TULISALO, Jukka, 04400 Järvenpää (FI); WICKHOLM, Niko, 00940 Helsinki (FI); PIRTIMAA, Minni, 02600 Espoo (FI); ALAKURTTI, Sami, 01450 Vantaa (FI); YLI_KAUHAULUOMA, Jari, 00790 Helsinki (FI)
(74) Representative: Lindberg, Berndt Åke
(86) International application number: PCT/IB2012/054632
(87) International publication number: WO 2013/038312

(56) References cited:
- WO-A2-00/26174
- CA-A1- 2 705 902
- US-A1- 2003 073 858
- SAMOSHINA N F ET AL: "Synthesis of Glycosides of Lupane-Type Triterpene Acids", CHEMISTRY OF NATURAL COMPOUNDS, CONSULTANTS BUREAU, NEW YORK, NY, US, vol. 39, no. 6, 1 November 2003 (2003-11-01), pages 575-582, XP008149724, ISSN: 0009-3130, DOI: 10.1023/B:CONC.0000018113.79735.34
- DENISENKO, M. V. ET AL.: 'Oxidation of betulin, dihydrobetulin and 3-beta-28-dihydroxy-18-lupane by ruthenium tetraoxide' CHEM. NAT. COMP. 1991, pages 374 - 375, XP055148323
- HOLY, J. ET AL.: 'Dimethylaminopyridine derivatives of lupane triterpenoids are potent disruptors of mitocondrial structure and function' BIOORG. MED. CHEM. vol. 18, 2010, pages 6080 - 6088, XP027192851
- SHELDON, R. A. ET AL.: 'Green, catalytic oxidations of alcohols' ACC. CHEM. RES. vol. 35, 2002, pages 774 - 781, XP055040164
- BARTHEL, A. ET AL.: 'Oxidative transformations of betulinol' TETRAHEDRON vol. 64, 2008, pages 9225 - 9229, XP023904166
- MURRAY, A. ET AL.: 'Synthesis of FF-MAS from lithocholic acid' BIOORG. MED. CHEM. LETT. vol. 10, 2000, pages 1067 - 1068, XP004204607
- INOKUCHI, T. ET AL.: 'One-pot conversion of primary alcohols to alpha-oxygenated alkanals with TEMPO in combination with molecular oxygen and ruthenium complex' TET. LETT vol. 36, no. 18, 1995, pages 3223 - 3226, XP004028231

## Description

### Field of the invention

The invention relates to catalytic, environmentally benign oxidation of betulin leading to synthesis of betulinic acid.

### State of the art

Betulin having the structure 1 shown below is naturally occurring pentacyclic terpene alcohol of lupine family, also known as betulinol and lup-20(29)-eeni-3β,28-diol. Betulin is found in the bark of some tree species, particularly in the birch (*Betula* sp.) bark at best amounts up to 40% of the birch outer bark dry weight. In addition to betulin, also minor amounts of betulin derivatives are obtained from tree bark. There are known methods mainly based on extraction for isolation of betulin from bark material.

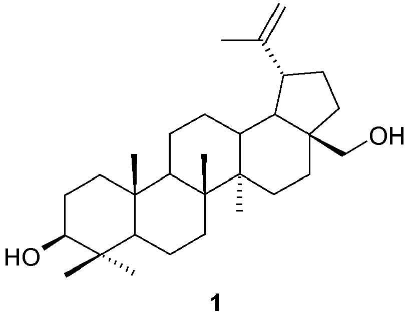

In some applications, poor solubility of betulin causes problems in respect to use and formulation, and accordingly, betulin is converted to its derivatives to improve solubility as well as bioactivity. In the production of said derivatives, reactivities of the functional groups of betulin, that is, primary and secondary groups and the double bond are typically utilized. Especially betulinic acid and betulonic acid seem to posses high bioactivities in several applications.

Suitability of betulin and the derivatives thereof for medical and cosmetic applications and for industrial chemical applications is known to some extent. Use of betulinic acid in cosmetic applications such as promoters of growth and as components in skin creams is known from WO 00/03749. The publication of WO 01/74327 discloses the use of betulinic acid in sun creams for prevention of detrimental effects of the UV-light. The publication of WO 2007/141389 discloses the use of betulonic acid in several cosmetic applications. The publication of WO 2007/141383 discloses the use of betulinic and betulonic acid as antifeedant agent for best management.

Betulinic acid having the structure of **3** shown in the scheme 1 below may be isolated e.g. from birch (*Betula* sp.) bark or cork of cork oak *(Quercus suber* L.) by extraction, and further may be produced by several methods mainly based on direct oxidation of the betulin or birch bark material. The reaction scheme below shows the direct oxidation of the betulin **1** according to US 5,804,575 as Jones oxidation in the presence of chromium(VI) oxide catalyst to give betu-Ionic acid **2,** followed by the selective reduction of betulonic acid 2 thus obtained to give betulinic acid **3.**

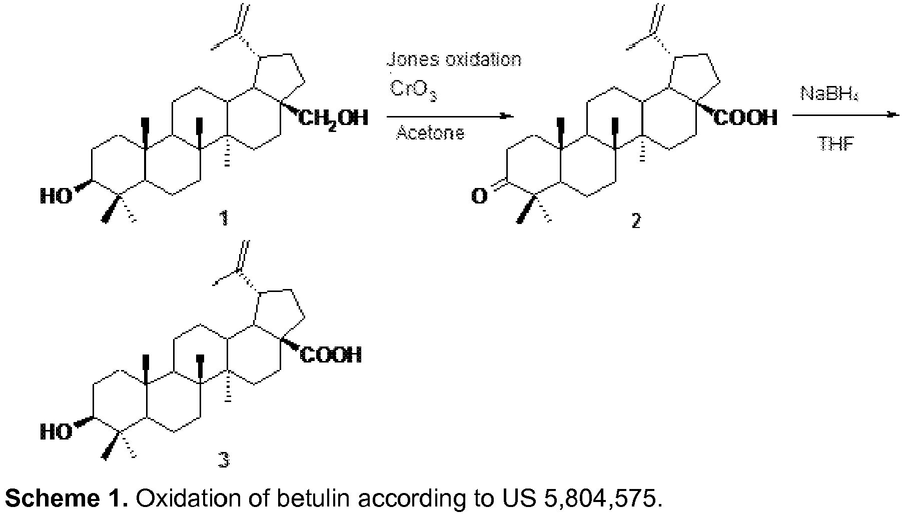

US-A-2003/073 858 discloses the oxidation of the 3-acetate of betulin to the corresponding aldehyde, using oxygen and Pd(OAc)₂, which is subsequently further oxidised to the the 3-acetate of betulinic acid, using oxygen and N-hydroxy-phthalimide (NHPI). In CA-A-2,705,902 betulin is converted to the corresponding aldehyde using TEMPO and N-chlorosuccinimide or TEMPO and trichloroiminocyanuric acid which is subsequently further oxidised to betulinic acid, using AgO.

The problem with the above method is that chromium (VI) oxide catalyst is carcinogenic and the reaction cumulates also large amounts of waste.

An alternative process for the production of betulinic acid is disclosed in US 2009/0076290 A1. The process shown below (scheme 2) includes contacting betulin with a composition that includes: a compound of formula **4,** dihydrogen potassium phosphate (KH₂PO₄) and diluted bleach with sodium chlorite (NaClO₂ for a period of time effective to provide betulinic aldehyde. Betulinic aldehyde is further oxidized to betulinic acid with sodium chlorite (NaClO₂) in the presence of dihydrogen potassium phosphate (KH₂PO₄).

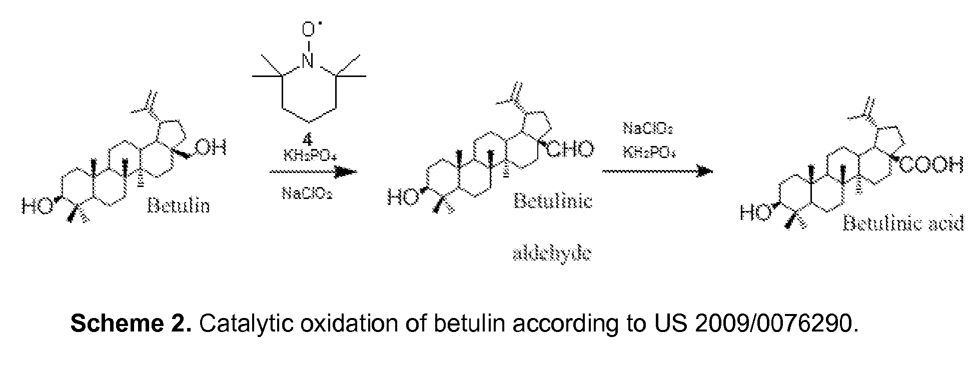

The problem with the above method is that the reaction time is long in the oxidation of betulin to betulinic aldehyde and the yield of betulinic aldehyde is not very high.

An alternative process for the production of betulinic acid is disclosed in US 5,804,575, comprising an oxidation step where 3-beta-hydroxyl of betulin is protected by acetylation. Isomerization and oxidation of secondary hydroxyl group of betulin is thus prevented.

### The objects of the invention

One object of the present invention is to develop environmentally benign catalytic method for oxidation of betulin to betulinic and/or betulonic aldehyde, and further converting the betulinic and/or betulonic aldehyde to betulinic acid.

Another object of the present invention is to develop an oxidation process for oxidation of betulin to betulinic and/or betulonic aldehyde, and further converting the betulinic and/or betulonic aldehyde to betulinic acid with a good yield and effective conversion.

Another object of the present invention is to develop an oxidation process for oxidation of betulin to betulinic and/or betulonic aldehyde, with catalyst and oxidant that are readily available and relatively cost effective, and further converting the betulinic and/or betulonic aldehyde to betulinic acid.

Another object of the present invention is to develop a synthesizing method for betulinic acid which would require as few process stages as possible.

### Description

The above mentioned objects can be achieved by the methods according to claims 1 and 16.

When trying to solve problems related to prior art, the inventors have now found that in contrast to existing methods the present invention introduces a method for oxidizing betulin to betulinic and/or betulonic aldehyde in an environmentally benign catalytic method with a good yield and effective conversion, and further converting the betulinic and/or betulonic aldehyde to betulinic acid.

The invention is based on the idea that betulin is oxidized to betulinic and/or betulonic aldehyde with a catalyst that is environmentally friendly, compared to for example chromium(VI) oxide, in the presence of an oxidant, and further converting betulinic and/or betulonic aldehyde to betulinic acid.

The method according to the present invention relates more precisely to a method according to claim 1. In said method betulinic acid is prepared from betulin by a process comprising oxidizing betulin to betulinic aldehyde with a ruthenium based catalyst, most preferably a ruthenium (II) or a ruthenium (VII) based catalyst, in the presence of an oxidant, the oxidant preferably being dioxygen, and further oxidizing the betulinic aldehyde to betulinic acid.

The present invention provides an environmentally benign catalytic method for oxidation of betulin to betulinic and/or betulonic aldehyde and further converting the betulinic and/or betulonic aldehyde to betulinic acid. In the oxidation of betulin to betulinic and/or betulonic aldehyde the catalyst and the oxidant are environmentally friendly, compared to for example chromium(VI) oxide catalyst which is carcinogenic. Due to a selective conversion and the possibility to regenerate the ruthenium based catalyst the reaction is environmentally benign. Furthermore, the catalyst and the oxidant of the present invention are readily available and relatively cost effective.

The present invention also provides an oxidation process for oxidation of betulin to betulinic and/or betulonic aldehyde and further converting the betulinic and/or betulonic aldehyde to betulinic acid with a good yield and effective conversion.

The claimed methods relates to preparation of betulinic acid from betulin characterized in that the method comprises oxidizing betulin to betulinic and/or betulonic aldehyde with ruthenium based catalyst catalyzed oxidation process in the presence of an oxidant and further converting the betulinic and/or betu-Ionic aldehyde to betulinic acid.

The catalytic oxidation of betulin to betulinic aldehyde and further to betulinic acid according to present invention proceeds according to scheme 3. Oxidation of betulin to betulinic aldehyde (reaction I) is performed in the presence of ruthenium based catalyst and an oxidant. Optionally a cocatalyst can be present in the oxidation of betulin to betulinic aldehyde. Oxidation of betulinic aldehyde to betulinic acid (reaction II) can be performed by any method known from the literature.

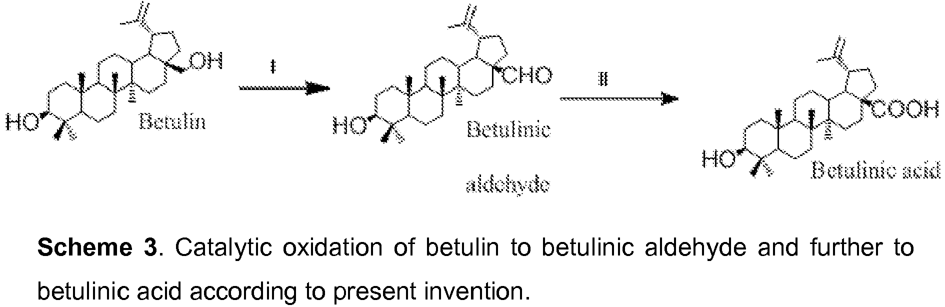

Oxidation of betulin to betulinic aldehyde is performed in the presence of ruthenium based catalyst. The ruthenium based catalyst can be any ruthenium based catalyst that is capable of catalyzing the oxidation reaction.

In one embodiment of the present invention said ruthenium based catalyst is represented by the formula

RuₘL_{m*n}

wherein Ru is ruthenium, L represents ligand, m is integer 1, 2 or 3 and n is integer from 0, 1, 2, 3, 4, 5 or 6. That is, one ruthenium molecule can have maximum of six ligands.

The ligand(s) of the ruthenium based catalyst are selected so that the ruthenium based catalyst is capable of catalyzing the oxidation reaction of betulin to betulinic aldehyde. The ligand(s) can be monodentate and/or polydentate.

Examples of monodentate ligands are, but not limited to, H₂O, ROH, NR₃, RCN, OH⁻, OOH⁻, RS⁻, RO⁻, RCOO⁻, OCN⁻, SCN⁻, N₃⁻, CN⁻, F⁻, Cl⁻, Br⁻, I⁻, O₂⁻_{,} NO3⁻, NO₂⁻, SO₄²⁻, SO₃²⁻, PO₄³⁻, organic phosphates, organic phosphonates, organic sulfates, organic sulfonates, and aromatic N donors such as pyridines, pyrazines, pyrazoles, imidazoles, benzimidazoles, pyrimidines, triazoles and thiazoles, with R being H, optionally substituted alkyl or optionally substituted aryl. Specific examples of monodentate ligands are phenolate, acetate and the like.

Examples of polydentate ligands are, but not limited to, Schiff base ligands and macropolycyclic rigid ligands. Specific examples of Schiff base ligands are disclosed for example in publication Viswanathamurthi P, Karvembu R, V Tharaneeswaran V, Natarajan K, J. Chem. Sci., 117, 235-238. Specific examples of macropolycyclic rigid ligands are disclosed for example in publication US 2003/017941 A1. Another specific example of polydentate ligands are N,N'-Ethylenebis(salicylimine) (salen ligand, having CAS number 94-93-9) and its derivatives.

The ruthenium based catalysts are ruthenium(II) compounds or ruthenium(VII) compounds. The Roman numeral in the parenthesis refers to oxidation number of ruthenium. A specific example of ruthenium(II) compound is dichlorotris(triphenylphosphine)ruthenium(II) (RuCl₂(PPh₃)₃). A specific example of ruthenium(VII) compound is tetra-n-propylammoniumperruthenate (TPAP).

Oxidation of betulin to betulinic aldehyde is carried out in the presence of an oxidant. The oxidant should be capable of oxidizing the primary alcohol of betulin in the presence of the ruthenium based. The oxidant is dioxygen originating from atmospheric oxygen. Oxygen dissolving to solvent system during the oxidation process can be facilitated by stirring.

The oxidation of betulin to betulinic aldehyde can be carried out in a solvent or mixture of solvents. Many common organic solvents and solvent combinations can be chosen and applied. The solvent is selected so that betulin is soluble therein. In a preferred embodiment the solvent is an inert solvent, that is, it does not react essentially with betulin or its derivatives or reactants. Examples of such inert solvents are aromatic hydrocarbons, saturated or unsaturated aliphatic hydrocarbons, branched or unbranched aliphatic hydrocarbons, ethers, halogenated aliphatic hydrocarbons, fluorous solvents, esters, ketones, H₂O and mixtures thereof. Most preferably the solvent is dichloromethane (CH₂Cl₂), dimethylformamide (DMF), tetrahydrofurane (THF), dimethyl sulfoxide (DMSO), perfluoroalkane, toluene, xylene or mixtures thereof.

By controlling the temperature of oxidation of betulin to betulinic aldehyde reaction time, conversion and side reactions can be affected. At very low temperatures conversion is poor and reaction times are long. At very high temperatures the oxidation process is difficult to control and more side reactions take place. The temperature during the oxidation of betulin to betulinic aldehyde with is from about 0 to about 150°C, preferably from about 0 to about 130 °C.

Pressure can be optionally applied to the oxidation of betulin to betulinic aldehyde. By applying pressure the oxidation occurs faster and with better conversion. The oxidation of betulin to betulinic aldehyde can be carried out under pressure at from about 1 to about 50 bar, preferably at about from 1 to about 15 bar.

In the method of the present invention oxidation of betulin to betulinic aldehyde can be affected by varying amounts of the ruthenium based catalyst. By increasing the amount of the ruthenium based catalyst the yield and conversion increases. The amount of the ruthenium based catalyst in the oxidation reaction can be about 1 to about 30 mole percent, relative to the betulin.

Optionally a cocatalyst can be present in the oxidation of betulin to betulinic aldehyde. The optional cocatalyst increases the yield and conversion. The optional cocatalyst is represented by the formula (5) 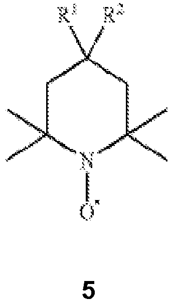 wherein each of R¹ and R² is independently hydrogen, alkyl, alkenyl, alkoxy, halo, haloalkyl, hydroxy, hydroxyalkyl, aryl, heteroaryl, heterocycle, cycloalkyl, alkanoyl, alkoxycarbonyl, amino, imino, alkylamino, acylamino, nitro, trifluoromethyl, trifluoromethoxy, carboxy, carboxyalkyl, keto, thioxo, alkylthio, alkylsulfinyl, alkylsulfonyl, cyano, carboxyl, acetamido, acetoxy, acetyl, benzamido, benzenesulfinyl, benzenesulfonamido, benzenesulfonyl, benzenesulfonylamino, benzoyl, benzoylamino, benzoyloxy, benzyl, benzyloxy, benzyloxycarbonyl, benzylthio, carbamoyl, carbamate, isocyannato, sulfamoyl, sulfinamoyl, sulfino, sulfo, sulfoamino, thiosulfo, NR^{x}R^{y} or COOR^{x}, wherein each R^{x} and R^{y} are independently H, alkyl, alkenyl, aryl, heteroaryl, heterocycle, cycloalkyl or hydroxyl; wherein any alkyl group is optionally substituted on carbon with keto (=O); wherein any alkyl group is optionally interrupted with one or more non-peroxide oxy (-O-), thio (-S-), imino (-N(H)-), methylene dioxy (-OCH₂O-), carbonyl (-C(=O)-), carboxy (-C(=O)O-), carbonyldioxy (-OC(=O)O-), carboxylato (-OC(=O)-), imine (C=NH), sulfinyl (SO), sulfonyl (SO₂) or [SiO]x, wherein x is about 1-10,000; or R¹ and R² together are thioxo (=S) or keto (=O).

Preferably R¹ is hydrogen and R² is hydrogen, alkyl, alkoxy, haloalkyl, hydroxy, heterocycle, amino, alkylamino, cyano, carboxyl; wherein any alkyl group is optionally substituted on carbon with keto (=O); wherein any alkyl group is optionally interrupted with one or more imino (-N(H)-), carbonyl (-C(=O)-) or [SiO]x, wherein x is about 1-10,000; or R¹ and R² together are keto (=O).

The term "alkyl" refers to a branched or unbranched saturated hydrocarbon chain preferably having from 1 to 10 carbon atoms, preferably 1 to 6 carbon atoms, and more preferably from 1 to 4 carbon atoms. Examples are methyl (Me, -CH₃), ethyl (Et, -CH₂CH₃), 1-propyl (n-Pr, n-propyl, -CH₂CH₂CH₃), 2-propyl (i-Pr, i-propyl, -CH(CH₃)₂), 1-butyl (n-Bu, n-butyl, -CH₂CH₂CH₂CH₃), 2-methyl-1-propyl (1-Bu, i-butyl, -CH₂CH(CH₃)₂), 2-butyl (s-Bu, s-butyl,-CH(CH₃)CH₂CH₃), 2-methyl-2-propyl (t-Bu, t-butyl, -C(CH₃)₃), 1-pentyl (n-pentyl, -CH₂CH₂CH₂CH₂CH₃), 2-pentyl (-CH(CH₃)CH₂CH₂CH₃), 3-pentyl (-CH(CH₂CH₃)₂), 2-methyl-2-butyl (-C(CH₃)₂CH₂CH₃), 3-methyl-2-butyl (-CH(CH₃)CH(CH₃)₂), 3-methyl-1-butyl (-CH₂CH₂CH(CH₃)₂), 2-methyl-1-butyl (-CH₂CH(CH₃)CH₂CH₃), 1-hexyl (-CH₂CH₂CH₂CH₂CH₂CH₃), 2-hexyl (-CH(CH₃)CH₂CH₂CH₂CH₃), 3-hexyl (-CH(CH₂CH₃)(CH₂CH₂CH₃)), 2-methyl-2-pentyl (-C(CH₃)₂CH₂CH₂CH₃), 3-methyl-2-pentyl (-CH(CH₃)CH(CH₃)CH₂CH₃), 4-methyl-2-pentyl (-CH(CH₃)CH₂CH(CH₃)₂), 3-methyl-3-pentyl (-C(CH₃)(CH₂CH₃)₂), 2-methyl-3-pentyl (-CH(CH₂CH₃)CH(CH₃)₂), 2,3-dimethyl-2-butyl (-C(CH₃)₂CH(CH₃)₂), 3,3-dimethyl-2-butyl (-CH(CH₃)C(CH₃)₃. The alkyl can be unsubstituted or substituted.

The term "alkenyl" refers to a branched or unbranched partially unsaturated hydrocarbon chain (i.e. a carbon-carbon, sp² double bond) preferably having from 2 to 10 carbon atoms, preferably 2 to 6 carbon atoms, and more preferably from 2 to 4 carbon atoms. Examples include, but are not limited to, ethylene or vinyl (-CH-CH₂), allyl (-CH₂CH-CH₂), cyclopentenyl (-C₅H₇), and 5-hexenyl (-CH₂ CH₂CH₂CH₂CH-CH₂). The alkenyl can be unsubstituted or substituted.

The term "alkoxy" refers to the groups alkyl-O-, where alkyl is defined herein. Preferred alkoxy groups include, e.g., methoxy, ethoxy, n-propoxy, iso-propoxy, n-butoxy, tert-butoxy, sec-butoxy, n-pentoxy, n-hexoxy, 1,2-dimethylbutoxy, and the like. The alkoxy can be unsubstituted or substituted.

The term "aryl" refers to an unsaturated aromatic carbocyclic group of from 3 to 12 carbon atoms having a single ring (e.g., phenyl) or multiple condensed (fused) rings, wherein at least one ring is aromatic (e.g., naphthyl, dihydrophe-nanthrenyl, fluorenyl, or anthryl). The aryl can be unsubstituted or substituted.

The term "cycloalkyl" refers to cyclic alkyl groups of from 3 to 10 carbon atoms having a single cyclic ring or multiple condensed rings. Such cycloalkyl groups include, by way of example, single ring structures such as cyclopropyl, cyclobutyl, cyclopentyl, cyclooctyl, and the like, or multiple ring structures such as adamantanyl, and the like. The cycloalkyl can be unsubstituted or substituted.

The term "halo" refers to fluoro, chloro, bromo, and iodo. Similarly, the term "halogen" refers to fluorine, chlorine, bromine, and iodine.

"Haloalkyl" refers to alkyl as defined herein substituted by 1-4 halo groups as defined herein, which may be the same or different. Representative haloalkyl groups include, by way of example, trifluoromethyl, 3-fluorododecyl, 12,12,12-trifluorododecyl, 2-bromooctyl, 3-bromo-6-chloroheptyl, and the like.

The term "heteroaryl" is defined herein as a monocyclic, bicyclic, or tricyclic ring system containing one, two, or three aromatic rings and containing at least one nitrogen, oxygen, or sulfur atom in an aromatic ring, and which can be unsubstituted or substituted, for example, with one or more, and in particular one to three, substituents, selected from alkyl, alkenyl, alkynyl, alkoxy, halo, haloalkyl, hydroxy, hydroxyalkyl, aryl, heterocycle, cycloalkyl, alkanoyl, alkoxycarbonyl, amino, alkylamino, dialkylamino, trifluoromethylthio, difluoromethyl, acylamino, nitro, trifluoromethyl, trifluoromethoxy, carboxy, carboxyalkyl, keto, thioxo, alkylthio, alkylsulfinyl, alkylsulfonyl and cyano. Examples of heteroaryl groups include, but are not limited to, 2H-pyrrolyl, 3H-indolyl, 4H-quinolizinyl, 4nH-carbazolyl, acridinyl, benzo[b]thienyl, benzothiazolyl, [beta]-carbolinyl, carbazolyl, chromenyl, cinnolinyl, dibenzo[b,d]furanyl, furazanyl, furyl, imidazolyl, imidizolyl, indazolyl, indolisinyl, indolyl, isobenzofuranyl, isoindolyl, isoquinolyl, isothiazolyl, isoxazolyl, naphthyridinyl, naptho[2,3-b], oxazolyl, perimidinyl, phenanthridinyl, phenanthrolinyl, phenarsazinyl, phenazinyl, phenothiazinyl, phenoxathiinyl, phenoxazinyl, phthalazinyl, pteridinyl, purinyl, pyranyl, pyrazinyl, pyrazolyl, pyridazinyl, pyridyl, pyrimidinyl, pyrimidinyl, pyrrolyl, quinazolinyl, quinolyl, quinoxalinyl, thiadiazolyl, thianthrenyl, thiazolyl, thienyl, triazolyl, and xanthenyl. In one embodiment the term "heteroaryl" denotes a monocyclic aromatic ring containing five or six ring atoms containing carbon and 1, 2, 3, or 4 heteroatoms independently selected from the group non-peroxide oxygen, sulfur, and N(Z) wherein Z is absent or is H, O, alkyl, phenyl or benzyl. In another embodiment heteroaryl denotes an ortho-fused bicyclic heterocycle of about eight to ten ring atoms derived therefrom, particularly a benz-derivative or one derived by fusing a propylene, or tetramethylene diradical thereto.

"Heterocycle" as used herein includes by way of example and not limitation those heterocycles described in Paquette, Leo A.; Principles of Modern Heterocyclic Chemistry (W.A. Benjamin, New York, 1968), particularly Chapters 1, 3, 4, 6, 7, and 9; The Chemistry of Heterocyclic Compounds, A Series of Monographs" (John Wiley & Sons, New York, 1950 to present), in particular Volumes 13, 14, 16, 19, and 28; and J. Am. Chem. Soc. (1960) 82:5566. In one specific embodiment of the invention "heterocycle" includes a "carbocycle" as defined herein, wherein one or more (e.g. 1, 2, 3, or 4) carbon atoms have been replaced with a heteroatom (e.g. O, N, or S).

Examples of heterocycles include by way of example and not limitation pyridyl, dihydroypyridyl, tetrahydropyridyl (piperidyl), thiazolyl, tetrahydrothiophenyl, sulfur oxidized tetrahydrothiophenyl, pyrimidinyl, furanyl, thienyl, pyrrolyl, pyrazolyl, imidazolyl, tetrazolyl, benzofuranyl, thianaphthalenyl, indolyl, indolenyl, quinolinyl, isoquinolinyl, benzimidazolyl, piperidinyl, 4-piperidonyl, pyrrolidinyl, 2-pyrrolidonyl, pyrrolinyl, tetrahydrofuranyl, tetrahydroquinolinyl, tetrahydroisoquinolinyl, decahydroquinolinyl, octahydroisoquinolinyl, azocinyl, triazinyl, 6H-1,2,5-thiadiazinyl, 2H,6H-1,5,2-dithiazinyl, thienyl, thianthrenyl, pyranyl, isobenzofuranyl, chromenyl, xanthenyl, phenoxathinyl, 2H-pyrrolyl, isothiazolyl, isoxazolyl, pyrazinyl, pyridazinyl, indolizinyl, isoindolyl, 3H-indolyl, 1 H-indazoly, purinyl, 4H-quinolizinyl, phthalazinyl, naphthyridinyl, quinoxalinyl, quinazolinyl, cinnolinyl, pteridinyl, 4aH-carbazolyl, carbazolyl, [beta]-carbolinyl, phenanthridinyl, acridinyl, pyrimidinyl, phenanthrolinyl, phenazinyl, phenothiazinyl, furazanyl, phenoxazinyl, isochromanyl, chromanyl, imidazolidinyl, imidazolinyl, pyrazolidinyl, pyrazolinyl, piperazinyl, indolinyl, isoindolinyl, quinuclidinyl, morpholinyl, oxazolidinyl, benzotriazolyl, benzisoxazolyl, oxindolyl, benzoxazolinyl, isatinoyl, and bis-tetrahydrofuranyl.

By way of example and not limitation, carbon bonded heterocycles are bonded at position 2, 3, 4, 5, or 6 of a pyridine, position 3, 4, 5, or 6 of a pyridazine, position 2, 4, 5, or 6 of a pyrimidine, position 2, 3, 5, or 6 of a pyrazine, position 2, 3, 4, or 5 of a furan, tetrahydrofuran, thiofuran, thiophene, pyrrole or tetrahydropyrrole, position 2, 4, or 5 of an oxazole, imidazole or thiazole, position 3, 4, or 5 of an isoxazole, pyrazole, or isothiazole, position 2 or 3 of an aziridine, position 2, 3, or 4 of an azetidine, position 2, 3, 4, 5, 6, 7, or 8 of a quinoline or position 1, 3, 4, 5, 6, 7, or 8 of an isoquinoline. Still more typically, carbon bonded heterocycles include 2-pyridyl, 3-pyridyl, 4-pyridyl, 5-pyridyl, 6-pyridyl, 3-pyridazinyl, 4-pyridazinyl, 5-pyridazinyl, 6-pyridazinyl, 2-pyrimidinyl, 4-pyrimidinyl, 5-pyrimidinyl, 6-pyrimidinyl, 2-pyrazinyl, 3-pyrazinyl, 5-pyrazinyl, 6-pyrazinyl, 2-thiazolyl, 4-thiazolyl, or 5-thiazolyl.

By way of example and not limitation, nitrogen bonded heterocycles are bonded at position 1 of an aziridine, azetidine, pyrrole, pyrrolidine, 2-pyrroline, 3-pyrroline, imidazole, imidazolidine, 2-imidazoline, 3-imidazoline, pyrazole, pyrazoline, 2-pyrazoline, 3-pyrazoline, piperidine, piperazine, indole, indoline, 1 H-indazole, position 2 of a isoindole, or isoindoline, position 4 of a morpholine, and position 9 of a carbazole, or [beta]-carboline. Still more typically, nitrogen bonded heterocycles include 1-aziridyl, 1-azetedyl, 1-pyrrolyl, 1-imidazolyl, 1-pyrazolyl, and 1-piperidinyl.

"Carbocycle" refers to a saturated, unsaturated or aromatic ring having 3 to 7 carbon atoms as a monocycle, 7 to 12 carbon atoms as a bicycle, and up to about 30 carbon atoms as a polycycle. Monocyclic carbocycles have 3 to 6 ring atoms, still more typically 5 or 6 ring atoms. Bicyclic carbocycles have 7 to 12 ring atoms, e.g., arranged as a bicyclo [4,5], [5,5], [5,6] or [6,6] system, or 9 or 10 ring atoms arranged as a bicyclo [5,6] or [6,6] system. Examples of carbocycles include cyclopropyl, cyclobutyl, cyclopentyl, 1-cyclopent-1-enyl, 1-cyclopent-2-enyl, 1-cyclopent-3-enyl, cyclohexyl, 1-cyclohex-1-enyl, 1-cyclohex-2-enyl, 1-cyclohex-3-enyl, phenyl, spiryl and naphthyl.

The term "alkanoyl" refers to C(-O)R, wherein R is an alkyl group as previously defined.

The term "alkoxycarbonyl" refers to C(-O)OR, wherein R is an alkyl group as previously defined.

The term "amino" refers to -NH₂, and the term "alkylamino" refers to -NR₂, wherein at least one R is alkyl and the second R is alkyl or hydrogen. The term "acylamino" refers to RC(-O)N, wherein R is alkyl or aryl.

The term "nitro" refers to -NO₂.

The term "trifluoromethyl" refers to -CF₃.

The term "trifluoromethoxy" refers to -OCF₃.

The term "cyano" refers to -CN.

The term "hydroxy" refers to -OH.

As to any of the above groups, which contain one or more substituents, it is understood, of course, that such groups do not contain any substitution or substitution patterns which are sterically impractical and/or synthetically non-feasible. In addition, the compounds of this invention include all stereochemical isomers arising from the substitution of these compounds.

As used herein, "aldehyde" refers to the functional group -C(-O)H, or any compound that includes such a group.

As used herein, "nitroxyl radical" refers to functional group (N-O.) and to compounds that include such a group.

The optional cocatalyst of formula 5 is most preferably
2,2,6,6-tetramethylpiperidine-1-oxyl (TEMPO), having the CAS Registry No. of 2564-83-2;
4-(2-chloroacetamido)-2,2,6,6-tetramethyl-1-piperidinyloxy, free radical;
4-(2-bromoacetamido)-2,2,6,6-tetramethyl-1-piperidinyloxy, free radical. The CAS Registry Number is 24567-97-3;
4-(2-iodoacetamido)-2,2,6,6-tetramethylpiperidine 1-oxyl or 4-(2-iodoacetamido)-2,2,6,6-tetramethyl-1-piperidinyloxy, free radical. The CAS Registry Number is 25713-24-0;
4-cyano-2,2,6,6-tetramethylpiperidine 1-oxy. The CAS Registry Number is 38078-71-6;
4-maleimido-2,2,6,6-tetramethyl-1-piperidinyloxy, free radical. The CAS Registry Number is 15178-63-9;
4-methoxy-2,2,6,6-tetramethyl-1-piperidinyloxy, free radical. The CAS Registry Number is 95407-69-5;
4-Oxo-2,2,6,6-tetramethyl-1-piperidinyloxy, free radical. The CAS Registry Number is 2896-70-0;
2,2,6,6-Tetramethyl-1-piperinyloxy, free radical on silica gel;
4-Amino-2,2,6,6-tetramethylpiperidinyloxy, free radical. The CAS Registry Number is 14691-88-4:
4-carboxy-2,2,6,6-tetramethylpiperidine 1-oxyl;
4-carboxy-2,2,6,6-tetramethylpiperidinyloxy, free radical. The CAS Registry Number is 37149-18-1;
4-Acetamido-2,2,6,6-tetramethylpiperidine 1-oxyl. The CAS Registry Number is 14691-89-5;
4-(2-chloroacetamido)-2,2,6,6-tetramethylpiperidine 1-oxyl". The CAS Registry Number is 36775-23-2;
4-hydroxyl-2,2,6,6-tetramethylpiperidine-1-oxyl;
polymer-bound TEMPO. "Polymer bound TEMPO" refers to having a particle size 100-200 mesh, loading: 1.0 mmol/g, 1% cross-linked with divinylbenzene;
which is a compound of the formula 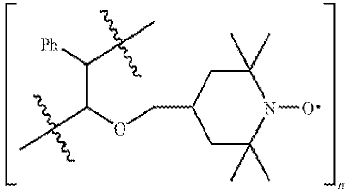 wherein the wavy lines indicate bonds of the polystyrene monomeric units to adjacent monomeric units.

Oxidation of betulinic aldehyde to betulinic acid can be performed by any know method in the literature. Oxidizing system in the oxidation of betulinic aldehyde to betulinic acid should selectively oxidize the aldehyde to the corresponding carboxylic acid.

There are many possible alternatives to be used as oxidizing systems in oxidation of betulinic aldehyde to betulinic acid. Such systems comprise appropriate oxidizing agent(s), possible buffer compound(s) and possible auxiliary agent(s).

Examples of suitable oxidizing agents are, but not limited to, dioxygen (O₂), sodium hypochlorite (NaOCI), sodium chlorite (NaClO₂) potassium chlorite (KClO₂), potassium hypochlorite (KOCI), peroxides such as hydrogen peroxide (H₂O₂), hypervalent iodine reagents (eg. 2-iodoxybenzoic acid (IBX), 2-iodoxybenzenesulfonic (IBS)), Oxone (2KHSO₅ KHSO₄ K₂SO₄) and phthalimides such as *N*-hydroxyphthalimide and mixtures thereof. As to other possible oxidizing agents we refer here to literature of this field.

In combination with above mentioned oxidizing agents it is often useful to use suitable buffer in an oxidation system. Examples of these buffers are potassium dihydrogen phosphate (KH₂PO₄) and sodium dihydrogen phosphate (NaH₂PO₄). pH-ranges for the above mentioned buffers are KH₂PO₄: pH 5.8 - 8; NaH₂PO4 pH 6 - 7.5. As to other possible buffers we refer here to literature of this field.

Auxiliary agents can be oxidation preventative agents which will prevent undesirable oxidation of betulin or its derivatives. Oxidation preventative agents are well known in this field and they are usually compounds, which will itself become reversible oxidazed. Examples of an oxidation preventative agent are 2-methyl-2-butene or tert.-butanol. The auxiliary agents can also be (ruthenium) scavenging agents such as QuadraPure Mercaptophenylaminobut-2-enoate ester. As to other possible auxiliary agents we refer here to literature of this field.

The oxidation of betulinic aldehyde to betulinic acid can be performed for example with oxidation system comprising NaOCl₂, NaH₂PO₄ and 2-methylbutene. Another example of suitable oxidation system is disclosed in US 2009/0076290 A1, where betulinic aldehyde is oxidized to betulinic acid with oxidation system comprising NaClO₂, KH₂PO₄ and 2-methyl-2-butene.

An alternative route for synthesis of betulinic acid as described herein for the purposes of comparison is presented in scheme 4 below. The alternative synthesis route follows steps la-III-IV. Oxidation of betulin to betulonic aldehyde (reaction Ia) is performed according to present invention. In reaction la the ruthenium based catalyst is TPAP and the oxidant is NMO. The betulonic aldehyde is oxidized to betulonic acid (reaction III), for example with an oxidation system comprising NaOCl₂, KH₂PO₄ and 2-methylbutene. The betulonic acid is reduced to betulinic acid (reaction IV), for example with sodiumborohydride (NaBH₄). Reactions III and IV can be performed by any known method in the literature.

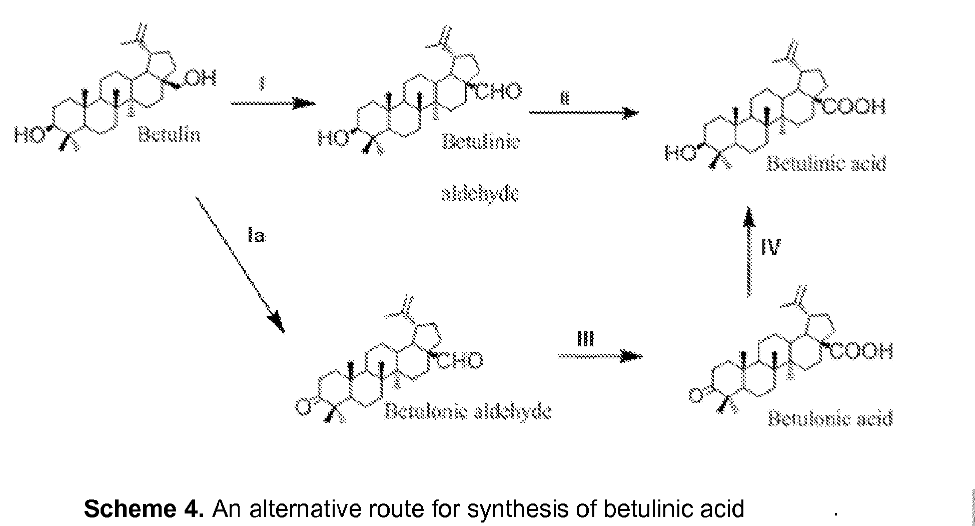

According to another embodiment of the present invention the betulin is first oxidized with a ruthenium based catalyst and air to betulinic aldehyde and the obtained betulinic aldehyde is oxidized without purification further to betulinic acid by using additional oxidation agent. In practice the catalytic oxidation of betulin and oxidation of betulinic aldehyde to corresponding acid happen simultaneously, in one pot (at the same vessel).

Above mentioned so called "one one pot oxidation method" comprises following process stages:
(a) oxidizing betulin to betulinic aldehyde in the presence of a ruthenium based catalyst and an oxidant,
(b) oxidizing said betulinic aldehyde to betulinic acid with an oxidizing system.

In stage (a) catalytic oxidation of betulin is performed by a ruthenium based catalyst in the presence of dioxygen. The catalytic oxidation reaction of betulin to betulinic aldehyde in stage (a) and oxidizing of betulinic aldehyde to betulinic acid (stage b) are performed simultaneously, in the same reaction vessel.

Because in this "one pot oxidation method" obtained betulinic aldehyde is not isolated but used as such, this method has an advantage of being effective and economic method for preparation betulinic acid from betulin. This embodiment of the inventive method may be suitable for a large scale conversion of betulin to betulinic acid or betulonic acid.

Neither above mentioned general methods (reaction routes I and II, and la, III and IV) for synthesizing betulinic acid nor above mentioned so called "one pot method" needs functional groups of betulin to be protected with protecting groups. Therefore neither cleavage of the protection groups is needed. Thus, reaction steps are reduced.

Optionally the ruthenium based catalyst can be supported by any conventional catalyst support such as zeolite, alumina, carbon, or the like.

### Examples

In scheme 5 is disclosed catalytic oxidation of betulin to betulinic aldehyde and further to betulinic acid according to the present invention. Reaction I is performed in the presence of ruthenium based catalyst and an oxidant. Optionally a cocatalyst can be present in the oxidation of betulin to betulinic aldehyde. Oxidation of betulinic aldehyde to betulinic acid (reaction II) can be performed by any known method in the literature.

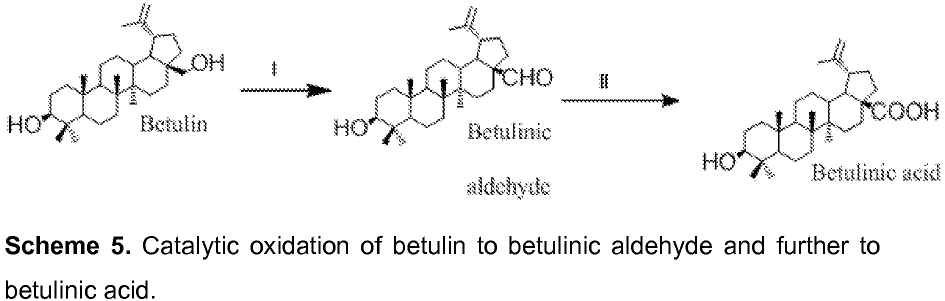

In table 1 are presented results of catalytic oxidation of betulin to betulinic aldehyde according to present invention (reaction I).

**Table 1. Catalytic oxidation of betulin to betulinic aldehyde according to present invention (reaction I).**

| **Example** | **Betulin (mg)** | **Solvent (ml)** | **Catalyst (mg)** | **Optional Cocatalyst (mg)** | **T (°C)** | **Pressure (bar)** | **Oxidant** | **Time (h)** | **Conversion (%)** | **Betulinic aldehyde (%)** | **Betulonic aldehyde (%)** | **Others (%)** |
|---|---|---|---|---|---|---|---|---|---|---|---|---|
| 1.1 | 220 | Toluene / 40 ml | RuCl₂(PPh₃)₃ / 20mg | TEMPO / 10 mg | 105 | 1 | O₂ balloon | 27 | 17 | 15 | - | 2 |
| 1.2 | 510 | Toluene / 50 ml | RuCl₂(PPh₃)₃ / 41 mg | TEMPO / 22mg | 100 | 4 | Air flow 20ml/min | 14 | 55 | 52 | 2 | 2 |
| 1.3 | 501 | Toluene / 50 ml | RuCl₂(PPh₃)₃ / 40 mg | TEMPO / 20 mg | 100 | 8 | Air flow 20ml/min | 14 | 54 | 50 | 2 | 2 |
| 1.4 | 503 | Toluene / 50 ml | RuCl₂(PPh₃)₃ / 41 mg | TEMPO / 41 mg | 100 | 8 | Air flow 20ml/min | 14 | 64 | 59 | 2 | 2 |
| 1.5 | 504 | Toluene / 50 ml | RuCl₂(PPh₃)₃ / 81 mg | TEMPO / 40 mg | 100 | 8 | Air flow 20ml/min | 14 | 74 | 69 | 3 | 2 |
| 1.6 | 1500 | Toluene / 90 ml | RuCl₂(PPh₃)₃ / 480 mg | TEMPO / 240 mg | 100 | 8 | Air flow 20ml/min | 7 | 99 | 80 | 1 | 18 |
| 1.7 | 503 | Toluene / 50 ml | TPAP / 39 mg | - | 110 | 1 | Air flow 50ml/min | 7 | 59 | 38 | 14 | 8 |
| 1.8 | 501 | 1,2-Dichloroethane / 50ml | TPAP / 40 mg | - | 80 | 1 | Air flow 50ml/min | 7 | 43 | 37 | 2 | 4 |
| 1.9 | 500 | Toluene / 50 ml | TPAP / 40 mg | - | 110 | 1 | Air flow 50ml/min | 7 | 48 | 30 | 10 | 8 |
| 1.10 | 221 | DCM / 15 ml | TPAP / 18 mg | - | 22 | 1 | O₂ balloon | 3 | 90 | 60 | 25 | 5 |
| *3.10 | 221 | DCM / 15 ml | TPAP / 17 mg | - | 22 | 1 | NMO / 204 mg = 3 eq | 2,5 | >95 | - | 95 | <1 |

| | | | | | | | | | | | | |
|---|---|---|---|---|---|---|---|---|---|---|---|---|
| *Reaction la | | | | | | | | | | | | |

### Reaction I

### Example 1.1

**Betulinic aldehyde:** Betulin (221 mg, 0,50 mmol), RuCl₂(PPh₃)₃ (20,2 mg, 0,021 mmol) and TEMPO (9,8 mg, 0,06272 mmol) in toluene (40 ml) was stirred under O₂ at 105°C for 24 hours. Reaction mixture was filtered through a pad of aluminium oxide, which was washed with toluene (150 ml) and dichloromethane (2x200 ml). Solvent was evaporated and residue was dried in a vacuum oven to yield betulinic aldehyde raw product 207 mg. GC purity analysis: betulin 83%, betulinic aldehyde 15%, others 2%.

### Example 1.5

**Betulinic aldehyde:** Betulin (500 mg, 1,1 mmol), RuCl₂(PPh₃)₃ (80 mg, 0,08 mmol) and TEMPO (40 mg, 0,26 mmol) in toluene (50 ml) was stirred at 8 atm air pressure, with 20 ml/min air flow, for 14 hours at 100 °C to yield betulinic aldehyde as main product. GC purity analysis: betulin 26%, betulinic aldehyde 69%, betulonic aldehyde 3%, others 2%.

### Example 1.10

**Betulinic aldehyde:** Betulin (221 mg, 0,50 mmol), 4A molecular sieves (504 mg) in dry dichloromethane (15 ml) was stirred under O₂ atmosphere for 30 min. TPAP (18,1 mg, 0,052 mmol) was added and reaction mixture was stirred under O₂ for 160 min at room temperature. Reaction mixture was filtered through pad of silica gel, which was washed with EtOAc (180 ml). Solvent was evaporated and residue was dried in vacuum oven to yield betulinic aldehyde raw product 235 mg. GC purity analysis: betulin 10%, betulinic aldehyde 60%, betulonic aldehyde 25%, others 5%.
¹H NMR (300 MHz, CDCl₃) 0.76 (s, 3H), 0.84 (s, 3H), 0.91 (s, 3H), 0.97 (s, 3H), 0.99 (s, 3H), 1.69 (s, 3H), 2.85 (m, 1 H), 3.17 (dd, 1 H), 4.63 (s, 1 H), 4.76 (s, 1H), 9.68 (s, 1H).

In table 2 are presented results of oxidation of betulinic aldehyde to betulinic acid (reaction II).

**Table 2. Results of oxidation of betulinic aldehyde to betulinic acid (reaction II).**

| Example | Betulinic aldehyde (mg) | Oxidation system | Yield of betulinic acid (%) |
|---|---|---|---|
| 2.1 | 1000 | NHPI (N-hydroxyphthalimide) / 22 mg + Air | 48 |
| 2.3 | 1000 | NaOCl₂/800 mg + NaH₂PO₄ + 2-methyl butene | 68 |
| 2.4 | 1000 | NaOC1₂/1700mg + NaH₂PO₄ + 2-methylbutene | 76 |
| 2.5 | 650 | NaClO₂/1800mg + H₂O₂/ 2.0 ml (30%) + NaH₂PO₄ | 10 |
| 2.6 | 650 | NaClO₂/2700mg + NaH₂PO₄ + 2-methylbutene | 22 |

### Reaction II

### Example 2.1

**Betulinic acid:** Betulinic aldehyde (1,00 g, 2,3 mmol) and NHPI (22 mg) in toluene was stirred, with 100 ml/min air flow, for 2,5 hours at 3 °C to yield betulinic acid as main product (48 %).

### Example 2.3

**Betulinic acid:** Betulinic aldehyde (1,00 g, 2,3 mmol), NaOCl₂ (0,8 g) + NaH₂PO₄ (15 ml; 0,5 M) + 2-methylbutene (3 ml) + dimethyl dioctadecyl ammonium chloride (90 mg) in toluene (30 ml) was stirred for 20 hours at 21 °C to yield betulinic acid as main product (68 %).

### Example 2.4

### (comparative)

**Betulinic acid:** Betulinic aldehyde (1,00 g, 2,3 mmol), NaOCl₂ (1,7 g) + NaH₂PO₄ (20 ml; 0,8 M) + 2-methylbutene (3 ml) + tert-butanol (17 ml) in toluene (30 ml) was stirred for 22 hours at 21 °C to yield betulinic acid as main product (76 %).

In scheme 6 is disclosed catalytic oxidation of betulin to betulonic aldehyde and further to betulonic acid. The betulonic acid is reduced to betulinic acid. Reaction la is performed in the presence of ruthenium based catalyst TPAP and oxidant NMO according to present invention. Oxidation of betulonic aldehyde to betulonic acid (reaction III) and reduction of betulonic acid to betulinic acid (reaction IV) can be performed by any known method in the literature.

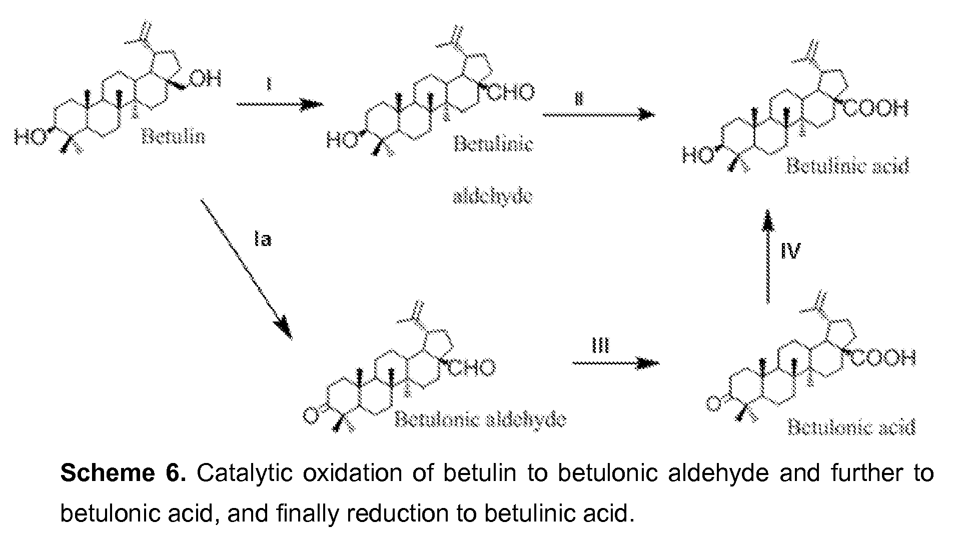

### Reaction Ia

### Example 3.10

### (comparative)

**Betulonic aldehyde:** Betulin (221 mg, 0,50 mmol), 4A molecular sieves (508 mg), NMO (204 mg, 1,51 mmol) in dry dichloromethane (15 ml) was stirred under argon for 30 min. TPAP (17,4 mg, 0,050 mmol) was added and reaction mixture was stirred for additional 120 min. Reaction mixture was filtered through pad of silica gel, which was washed with EtOAc (180 ml). Solvent was evaporated and residue was dried in a vacuum oven to yield betulonic aldehyde as main product (218 mg). GC purity analysis: betulin 4%, betulonic aldehyde 95%, others 1%.
¹H NMR (300 MHz, CDCl₃) 9,65; 4,74; 4,59; 2,85.
¹³C NMR (300 MHz, CDCl₃) 218,04; 206,51; 149,61; 110,18; 66,46; 54,96; 49,77; 47,90; 47,43; 42,56; 40,72; 39,58; 38,70; 36,84; 34,08; 33,57; 33,12; 29,08; 28,75; 26,78; 25,48; 21,23; 20,98; 19,59; 18,96; 15,93; 15,68; 14,14.

### Reaction III

**Betulonic acid:** Betulonic aldehyde (1,00 g, 2,3 mmol), NaOCl₂ (1,7 g) + NaH₂PO₄ (20 ml; 0,8 M) + 2-methylbutene (3 ml) + tert-butanol (17 ml) in toluene (30 ml) was stirred for 22 hours at 21 °C to yield betulonic acid as main product (76 %).

### Reaction IV

**Betulinic acid:** To a solution of betulonic acid (13 g, 2,3 mmol), water (130 ml), NaOH (26 ml, 5%) and 2-propanol (120 ml) was added NaBH₄ (1,2 g) and stirred for 3 hours. HCl solution (130 ml, 10%) was added and precipitate was filtered, washed with water (4*50 ml) and dried in a vacuum oven to yield betulinic acid 12,24 g (94%).

### The "one pot oxidation method"

### Example 1.6

**Betulinic aldehyde:** 150 ml reactor was loaded with betulin (1.5 g), RuCl₂PPh₃ (0.48 g), TEMPO (0.24 g) and toluene (90 ml). Reactor was pressurized to 8 bar and airflow was adjusted to 20 ml/min. Heating (set temp 100 ⁰C) and stirring (300 rpm) were started and continued for 7 hours to yield betulinic aldehyde as main product. CG purity analysis: 1 % betulin, 80 % betulinic aldehyde and others 19%. Obtained filtrate (including betulinic aldehyde 80%) was subsequently used in the next reaction step in one pot without further purification.

### Example 2.5

**Betulinic acid.** 30 ml filtrate (including 0.65 g of betulinic aldehyde, purity 80%) from example 1.6 was filtered into a 100 ml flask. 0.1 g NaH₂PO₄ in 5 ml H₂O and 0.5 ml 30 % H₂O₂ were added to the flask. Reaction mixture was stirred (300 rpm) in an ice-water bath and 0.6 g NaO₂Cl in 10 ml H₂O was added dropwise during 30 min and reaction mixture was stirred for 5 hours. Additional 0.6 g NaH₂PO₄ in 10 ml H₂O, 1.5 ml 30 % H₂O₂ and 1.2 g NaO₂Cl in 20 ml H₂O were added in two proportions to the flask after 5 and 17 hours from the start of the reaction. Reaction mixture was let to stir 6 more hours and CG sample was taken. GC showed that there were 32 % betulinic aldehyde, 10 % betulinic acid, 34 % unknown component 1 and 11 % unknown component 2.

### Example 2.6

**Betulinic acid.** 30 ml filtrate (including 0.65 g of betulinic aldehyde, purity 80%) from example 1.6 was filtered into a 100 ml flask. 8.5 ml t-BuOH and 5 ml 2-methyl 2-butene and 1.3 g NaH₂PO₄ in 15 ml H₂O were added to the flask. 0.9 g NaClO₂ in 10 ml H₂O was added to the flask dropwise during 30 minutes and reaction mixture was stirred for 5 hours. Additional 1.8 g NaClO₂ in 20 ml H₂O, 2.6 g NaH₂PO₄ in 30 ml H₂O and 4 ml 2-methyl 2-butene were added to in two proportions to the flask after 5 and 17 hours from the start of the reaction. Reaction mixture was let to stir 6 more hours and CG sample was taken. GC showed that there were 54 % betulinic aldehyde, 22 % betulinic acid, 5 % unknown component 3 and 8 % unknown component 4.

## Claims

1. A method for preparation of betulinic acid from betulin, **characterized in that** said method comprises oxidizing betulin to betulinic aldehyde with a ruthenium(II) or ruthenium(VII) based catalyst catalyzed oxidation process in the presence of an oxidant, the oxidant being dioxygen originating from atmospheric oxygen, and the betulinic aldehyde is oxidized to betulinic acid.

2. The method according to claim 1, **characterized in that** the ruthenium(II) or ruthenium(VII) based catalyst is dichlorotris(triphenylphosphine)ruthenium(ll) (RuCl₂(PPh₃)₃) or tetra-n-propylammoniumperruthenate (TPAP).

3. The method according to claim 1 or 2, **characterized in that** amount of the ruthenium(II) or ruthenium(VII) based catalyst is about 1 to about 30 mole percent, relative to the betulin.

4. The method according to any one of the previous claims, **characterized in that** oxidation of betulin to betulinic aldehyde is carried out in a solvent selected from the group consisting of aromatic hydrocarbons, saturated or unsaturated aliphatic hydrocarbons, branched or unbranched aliphatic hydrocarbons, ethers, halogenated aliphatic hydrocarbons, fluorous solvents, esters, ketones, H₂O and mixtures thereof.

5. The method according to claim 5, **characterized in that** solvent is CH₂Cl₂, DMF, THF, DMSO, perfluoroalkane, toluene, xylene or mixtures thereof.

6. The method according to any one of the previous claims, **characterized in that** temperature during the oxidation of betulin is from about 0 to about 150 °C.

7. The method according to any one of the previous claims, **characterized in that** oxidizing betulin to betulinic aldehyde is carried out under pressure from about 1 to about 50 bar, preferably from about 1 to about 15 bar.

8. The method according to any one of the previous claims, **characterized in that** oxygen dissolving to solvent system during the oxidation process is facilitated by stirring.

9. The method according to any one of the previous claims, **characterized in that** oxidizing betulin to betulinic aldehyde is carried out in the presence of a cocatalyst.

10. The method according to claim 9, **characterized in that** the cocatalyst is represented by the formula (5) 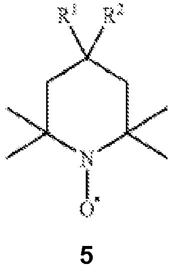 wherein each of R¹ and R² is independently hydrogen, alkyl, alkenyl, alkoxy, halo, haloalkyl, hydroxy, hydroxyalkyl, aryl, heteroaryl, heterocycle, cycloalkyl, alkanoyl, alkoxycarbonyl, amino, imino, alkylamino, acylamino, nitro, trifluoromethyl, trifluoromethoxy, carboxy, carboxyalkyl, keto, thioxo, alkylthio, alkylsulfinyl, alkylsulfonyl, cyano, carboxyl, acetamido, acetoxy, acetyl, benzamido, benzenesulfinyl, benzenesulfonamido, benzenesulfonyl, benzenesulfonylamino, benzoyl, benzoylamino, benzoyloxy, benzyl, benzyloxy, benzyloxycarbonyl, benzylthio, carbamoyl, carbamate, isocyannato, sulfamoyl, sulfinamoyl, sulfino, sulfo, sulfoamino, thiosulfo, NR^{x}R^{y} or COOR^{x}, wherein each R^{x} and R^{y} are independently H, alkyl, alkenyl, aryl, heteroaryl, heterocycle, cycloalkyl or hydroxyl; wherein any alkyl group is optionally substituted on carbon with keto (=O); wherein any alkyl group is optionally interrupted with one or more non-peroxide oxy (-O-), thio (-S-), imino (-N(H)-), methylene dioxy (-OCH₂O-), carbonyl (-C(=O)-), carboxy (-C(=O)O-), carbonyldioxy (-OC(=O)O-), carboxylato (-OC(=O)-), imine (C=NH), sulfinyl (SO), sulfonyl (SO₂) or [SiO]x, wherein x is about 1-10,000; or R¹ and R² together are thioxo (=S) or keto (=O).

11. The method according to claim 10, **characterized in that,** wherein R¹ is hydrogen and R² is hydrogen, alkyl, alkoxy, haloalkyl, hydroxy, heterocycle, amino, alkylamino, cyano, carboxyl; wherein any alkyl group is optionally substituted on carbon with keto (=O); wherein any alkyl group is optionally interrupted with one or more imino (-N(H)-), carbonyl (-C(=O)-) or [SiO]x, wherein x is about 1-10,000; or R¹ and R² together are keto (=O).

12. The method according to claims 10-11, **characterized in that** the compound of formula (5) is 2,2,6,6-tetramethylpiperidine 1-oxyl (TEMPO).

## Patentansprüche

1. Verfahren zur Herstellung von Betulinsäure aus Betulin, **dadurch gekennzeichnet, dass** das Verfahren das Oxidieren von Betulin zu Betulinaldehyd mittels eines Oxidationsverfahrens mit einem Katalysator auf Basis von Ruthenium(II) oder Ruthenium(VII) in Gegenwart eines Oxidationsmittels umfasst, wobei das Oxidationsmittel Disauerstoff ist, der aus atmosphärischem Sauerstoff stammt, und der Betulinaldehyd zu Betulinsäure oxidiert wird.

2. Verfahren nach Anspruch 1, **dadurch gekennzeichnet, dass** der Katalysator auf Basis von Ruthenium(II) oder Ruthenium(VII) Dichlortris(triphenylphosphin)ruthenium(II) (RuCl₂(PPh₃)₃) oder Tetra-n-propylammoniumperruthenat (TPAP) ist.

3. Verfahren nach Anspruch 1 oder 2, **dadurch gekennzeichnet, dass** die Menge des Katalysators auf Basis von Ruthenium(II) oder Ruthenium(VII), bezogen auf das Betulin, etwa 1 bis etwa 30 Molprozent beträgt.

4. Verfahren nach einem der vorhergehenden Ansprüche, **dadurch gekennzeichnet, dass** die Oxidation von Betulin zu Betulinaldehyd in einem Lösemittel durchgeführt wird, das ausgewählt ist aus der Gruppe, bestehend aus aromatischen Kohlenwasserstoffen, gesättigten oder ungesättigten aliphatischen Kohlenwasserstoffen, verzweigten oder unverzweigten aliphatischen Kohlenwasserstoffen, Ethern, halogenierten aliphatischen Kohlenwasserstoffen, fluorhaltigen Lösemitteln, Estern, Ketonen, H₂O und Gemischen davon.

5. Verfahren nach Anspruch 5, **dadurch gekennzeichnet, dass** das Lösemittel CH₂Cl₂, DMF, THF, DMSO, Perfluoralkan, Toluol, Xylol oder Gemische davon ist.

6. Verfahren nach einem der vorhergehenden Ansprüche, **dadurch gekennzeichnet, dass** die Temperatur während der Oxidation von Betulin etwa 0 bis etwa 150 °C beträgt.

7. Verfahren nach einem der vorhergehenden Ansprüche, **dadurch gekennzeichnet, dass** das Oxidieren von Betulin zu Betulinaldehyd unter einem Druck von etwa 1 bis etwa 50 bar, vorzugsweise von etwa 1 bis etwa 15 bar, durchgeführt wird.

8. Verfahren nach einem der vorhergehenden Ansprüche, **dadurch gekennzeichnet, dass** das Lösen von Sauerstoff zu dem Lösemittelsystem während des Oxidationsverfahrens durch Rühren erleichtert wird.

9. Verfahren nach einem der vorhergehenden Ansprüche, **dadurch gekennzeichnet, dass** das Oxidieren von Betulin zu Betulinaldehyd in Gegenwart eines Cokatalysators durchgeführt wird.

10. Verfahren nach Anspruch 9, **dadurch gekennzeichnet, dass** der Cokatalysator durch die Formel (5) dargestellt ist 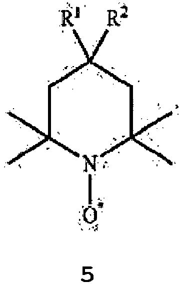 wobei jedes von R¹ und R² unabhängig Wasserstoff, Alkyl, Alkenyl, Alkoxy, Halogen, Halogenalkyl, Hydroxy, Hydroxyalkyl, Aryl, Heteroaryl, Heterocyclus, Cycloalkyl, Alkanoyl, Alkoxycarbonyl, Amino, Imino, Alkylamino, Acylamino, Nitro, Trifluormethyl, Trifluormethoxy, Carboxy, Carboxyalkyl, Keto, Thioxo, Alkylthio, Alkylsulfinyl, Alkylsulfonyl, Cyano, Carboxyl, Acetamido, Acetoxy, Acetyl, Benzamido, Benzolsulfinyl, Benzolsulfonamido, Benzolsulfonyl, Benzolsulfonylamino, Benzoyl, Benzoylamino, Benzoyloxy, Benzyl, Benzyloxy, Benzyloxycarbonyl, Benzylthio, Carbamoyl, Carbamat, Isocyanato, Sulfamoyl, Sulfinamoyl, Sulfino, Sulfo, Sulfoamino, Thiosulfo, NR^{x}R^{y} oder COOR^{x} ist, wobei jedes R^{x} und R^{y} unabhängig H, Alkyl, Alkenyl, Aryl, Heteroaryl, Heterocyclus, Cycloalkyl oder Hydroxyl sind; wobei eine beliebige Alkylgruppe wahlweise am Kohlenstoff mit Keto(=O) substituiert ist; wobei eine beliebige Alkylgruppe wahlweise durch ein oder mehrere Nichtperoxid-Oxy (-O-), Thio (-S-), Imino (-N(H)-), Methylendioxy (-OCH₂O-), Carbonyl (-C(=O)-), Carboxy (-C(=0)0-), Carbonyldioxy (-OC(=O)O-), Carboxylato (-OC(=O)-), Imin (C=NH), Sulfinyl (SO), Sulfonyl (SO₂) oder [SiO]x unterbrochen ist, wobei x etwa 1 bis 10.000 beträgt; oder R¹ und R² zusammen Thioxo (=S) oder Keto (=0) sind.

11. Verfahren nach Anspruch 10, **dadurch gekennzeichnet, dass** R¹ Wasserstoff ist und R² Wasserstoff, Alkyl, Alkoxy, Halogenalkyl, Hydroxy, Heterocyclus, Amino, Alkylamino, Cyano, Carboxyl ist; eine beliebige Alkylgruppe wahlweise an Kohlenstoff mit Keto (=0) substituiert ist; eine beliebige Alkylgruppe wahlweise durch ein oder mehrere Imino (-N(H)-), Carbonyl (-C(=0)-) oder [SiO]x unterbrochen ist, wobei x etwa 1 bis 10.000 beträgt; oder R¹ und R² zusammen Keto sind (=O).

12. Verfahren nach Anspruch 10 und 11, **dadurch gekennzeichnet, dass** die Verbindung mit der Formel (5) 2,2,6,6-Tetramethylpiperidin-1-oxyl (TEMPO) ist.

## Revendications

1. Procédé de préparation d'acide bétulinique à partir de bétuline, **caractérisé en ce que** ledit procédé comprend l'oxydation de la bétuline en aldéhyde bétulinique avec un procédé d'oxydation catalysé avec un catalyseur à base de ruthénium(II) ou de ruthénium(VII) en présence d'un oxydant, l'oxydant étant du dioxygène originaire de l'oxygène atmosphérique, et l'aldéhyde bétulinique est oxydé en acide bétulinique.

2. Procédé selon la revendication 1, **caractérisé en ce que** le catalyseur à base de ruthénium(II) ou de ruthénium(VII) est du dichlorotris(triphénylphosphine)ruthénium(II) (RuCl₂(PPh₃)₃) ou du tétra-n-propylammoniumperruthénate (TPAP).

3. Procédé selon la revendication 1 ou 2, **caractérisé en ce que** la quantité de catalyseur à base de ruthénium(II) ou de ruthénium(VII) est d'environ 1 à environ 30 mole pour cents, par rapport à la bétuline.

4. Procédé selon l'une quelconque des revendications précédentes, **caractérisé en ce que** l'oxydation de la bétuline en aldéhyde bétulinique est réalisée dans un solvant choisi dans le groupe constitué des hydrocarbures aromatiques, des hydrocarbures aliphatiques saturés ou non saturés, des hydrocarbures aliphatiques ramifiés ou non ramifiés, des éthers, des hydrocarbures aliphatiques halogénés, des solvants fluorés, des esters, des cétones, d'H₂O et des mélanges de ceux-ci.

5. Procédé selon la revendication 5, **caractérisé en ce que** le solvant est du CH₂Cl₂, du DMF, du THF, du DMSO, un perfluoroalcane, du toluène, du xylène ou des mélanges de ceux-ci.

6. Procédé selon l'une quelconque des revendications précédentes, **caractérisé en ce que** la température durant l'oxydation de la bétuline est d'environ 0 à environ 150 °C.

7. Procédé selon l'une quelconque des revendications précédentes, **caractérisé en ce que** l'oxydation de la bétuline en aldéhyde bétulinique est effectuée sous une pression d'environ 1 à environ 50 bar, de préférence d'environ 1 à environ 15 bar.

8. Procédé selon l'une quelconque des revendications précédentes, **caractérisé en ce que** la dissolution de l'oxygène dans le système de solvant durant le processus d'oxydation est facilitée par une agitation.

9. Procédé selon l'une quelconque des revendications précédentes, **caractérisé en ce que** l'oxydation de la bétuline en aldéhyde bétulinique est effectuée en présence d'un co-catalyseur.

10. Procédé selon la revendication 9, **caractérisé en ce que** le co-catalyseur est représenté par la formule (5) 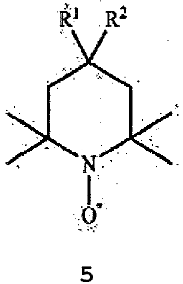 où chaque R¹ et R² est indépendamment un atome d'hydrogène, un groupe alkyle, alcényle, alcoxy, halo, haloalkyle, hydroxy, hydroxyalkyle, aryle, hétéroaryle, hétérocycle, cycloalkyle, alcanoyle, alcoxycarbonyle, amino, imino, alkylamino, acylamino, nitro, trifluoro-méthyle, trifluorométhoxy, carboxy, carboxyalkyle, céto, thioxo, alkylthio, alkyl-sulfinyle, alkylsulfonyle, cyano, carboxyle, acétamido, acétoxy, acétyle, benzamido, benzènesulfinyle, benzènesulfonamido, benzènesulfonyle, benzènesulfonylamino, benzoyle, benzoylamino, benzoyloxy, benzyle, benzyloxy, benzyloxycar- bonyle, benzylthio, carbamoyle, carbamate, isocyannato, sulfamoyle, sulfinamoyle, sulfino, sulfo, sulfoamino, thiosulfo, NR^{x}R^{y} ou COOR^{x}, où chaque R^{x} et R^{y} sont indépendamment un atome de H, un groupe alkyle, alcényle, aryle, hétéroaryle, hétérocycle, cycloalkyle ou hydroxyle ; où un groupe alkyle quelconque est éventuellement substitué sur l'atome de carbone avec un groupe céto(=O) ; où un groupe alkyle quelconque est éventuellement interrrompu avec un ou plusieurs groupes non peroxydes oxy (-0-), thio (-S-), imino (-N(H)-), méthylene dioxy (-OCH₂O-), carbonyle (-C(=O)-), carboxy (-C(=O)O-), carbonyldioxy (-OC(=O)O-), carboxylato (-OC(=O)-), imine (C=NH), sulfinyle (SO), sulfonyle (SO₂) ou [SiO]x, où x est environ 1 à 10 000 ; ou R¹ et R² ensemble sont un groupe thioxo (=S) ou céto (=O).

11. Procédé selon la revendication 10, **caractérisé en ce que,** où R¹ est un atome d'hydrogène et R² est un atome d'hydrogène, un groupe alkyle, alcoxy, haloalkyle, hydroxy, hétérocycle, amino, alkylamino, cyano, carboxyle ; où un groupe alkyle quelconque est éventuellement substitué sur l'atome de carbone avec un groupe céto (=O) ; où un groupe alkyle quelconque est éventuellement interrompu avec un plusieurs groupes imino (-N(H)-), carbonyle (-C(=O)-) ou [SiO]x, où x est environ 1 à 10 000 ; ou R¹ et R² ensemble sont un groupe céto (=O).

12. Procédé selon les revendications 10 à 11, **caractérisé en ce que** le composé de la formule (5) est du 2,2,6,6-tétraméthylpipéridine 1-oxyle (TEMPO).
